# EUROPEAN PATENT APPLICATION

(11) **EP 4 371 470 A1**
(43) Date of publication of application: **22.05.2024**
(21) Application number: 22208545.8
(22) Date of filing: 21.11.2022
(51) Int. Cl.: A61B 5/00, A61B 5/113, A61B 5/08

(54) **METHOD AND SYSTEM FOR ON/OFF WEARING DETECTION OF AN ACCELEROMETER-BASED WEARABLE DEVICE**

(71) Applicant: Koninklijke Philips N.V., 5656 AG Eindhoven (NL)
(72) Inventor: VERHAEGH, Wilhelmus Franciscus Johannes, 5656AG Eindhoven (NL); GARSSEN, Sjoerd, 5656AG Eindhoven (NL); VERNOOIJ, Carlijn Andrea, 5656AG Eindhoven (NL)
(74) Representative: Philips Intellectual Property & Standards

(57) **Abstract**

A system (10) includes a monitoring device (12) including an accelerometer (14), an on-board electronic processor (16), and a wireless transmitter or transceiver (18). The monitoring device configured to be attached to a patient and the accelerometer configured to measure accelerometer data (15). The on-board electronic processor of the monitoring device performs a health status monitoring method (100) including analyzing the accelerometer data to determine respiration rate data for the patient or an indication that respiration rate data cannot be determined from the accelerometer data; determining whether the monitoring device is attached to the patient based at least on the whether the analyzing determines that respiration rate data cannot be determined; and one of (i) displaying health status information for the patient generated from the accelerometer data and the determined respiration rate data; or (ii) displaying an indication (26) that the monitoring device is not attached to the patient.

## Description

The following relates generally to the patient monitoring arts, wearable medical device arts, accelerometer arts, respiration monitoring arts, heart rate monitoring arts, patient activity monitoring arts, and related arts.

### BACKGROUND

Health-related unobtrusive sensing systems enable replacement of continued hospitalization with obtrusive vital signs sensor technologies, centered around the individual, to provide remote monitoring of the subject's general health condition. Vital signs monitoring typically includes monitoring one or more of the following physical parameters: heart rate (HR), blood pressure (BP), respiratory rate (RR), core body temperature and blood oxygenation (SpO₂).

To assess a patient's condition or to determine a patient's degree of illness, a clinical decision support algorithm is often used, such as the spot-check-based early warning score (EWS) system in a general hospital ward. These algorithms take vital signs signals (or a subset thereof) measured of a patient and analyse trends or compare values to predefined thresholds of assumed normal values for EWS systems. The output of these algorithms informs clinicians on the assumed physiological instability of the patient based on which, combined with non-physiological factors, the clinician can assess whether changes are needed in the patient's treatment plan. While these algorithms are aimed at instability detection, they are sometimes used as indirect indicator of patient stability, and patient stability algorithms are also being developed.

By way of unobtrusive wearable vital signs monitoring systems, like vital sign patches, continuous collection of vital signs is made feasible in lower acuity care settings like the general ward and at home. These vital sign patches are often based on accelerometery signals, photoplethysmography (PPG) signals, electrocardiogram (ECG) measurements, bio-impedance signals, inductive magneto-electrogram signal, resistance, etc.

To reduce hospital costs, improve patient, and staff satisfaction while keeping healthcare outcomes high (or even improved), hospitals can employ remote monitoring of patients. In some examples, remote vital sign monitoring with a wearable sensor enables patients to be discharged home earlier after hospitalization while they are still being observed by a healthcare professional. The remote vital sign monitoring acquires HR, RR, and/or other vital sign samples continually (that is, on a frequent basis, e.g., every 5 minutes in a nonlimiting illustrative example). If, based on Clinical Decision System (CDS) scores computed using these vital sign readings, the patient shows signs of deterioration, then action should be taken. Examples are a phone or video call to confirm compliance with medication intake, making minor medication dose adjustments, re-assessment by a home care clinician visit, and if necessary, readmitting the patient to the hospital.

For reliability, the continuous remote monitoring should give accurate alarms or notifications if deterioration occurs. An overabundance of false alarms, where an alarm or notification is raised erroneously and the patient is doing well after all, leads to alarm fatigue. Alarm fatigue can be amongst the top challenges that hospitals face and can be seen as a risk of using sensors in healthcare. In particular, for wearable sensors, movement artifacts in wearable sensor data can also result in an overabundance of false alarms, leading to alarm fatigue.

The continuous remote monitoring should also be reliable in obtaining vital signs data from the patient. This refers to technical and technological reliability, but also to reliability that the patient is using and wearing the sensor accurately and sufficiently. If the patient is not wearing the sensor, no remote monitoring can happen. This is especially risky when the hospital does not notice the sensor has been taken off and is still relying on alarms to arrive in case of deterioration. This issue of patients inadvertently taking off the wearable sensor is also an important criterium for pharmaceutical companies to rely upon remote vital sign monitoring when conducting clinical trials. In another example, the wearable sensor can have a dead battery. In a clinical trial setting, a lack of patient compliance in wearing a sensor can have significant effects on the possibility of alarm generation and may adversely impact conclusions drawn from the clinical trial. Yet another use of such a method and system is for healthcare providers (e.g., hospitals and the like) using remote monitoring of patients undergoing treatment. In a similar way as for the pharmaceutical research, healthcare providers want to track whether the treatment has a sufficient positive effect or whether it needs any adjustment, for which reliable wearing is a precondition.

The following discloses certain improvements to overcome these problems and others.

### SUMMARY

In one aspect, a system includes a monitoring device including an accelerometer, an on-board electronic processor, and a wireless transmitter or transceiver. The monitoring device is configured to be attached to a patient and the accelerometer configured to measure accelerometer data. A clinical information system includes at least one electronic processor. The clinical information system is operatively connected with the monitoring device via the wireless transmitter or transceiver of the monitoring device. The on-board electronic processor of the monitoring device and the clinical information system cooperatively perform a health status monitoring method including analyzing the accelerometer data to determine respiration rate data for the patient or an indication that respiration rate data cannot be determined from the accelerometer data; determining whether the monitoring device is attached to the patient based at least on the whether the analyzing determines that respiration rate data cannot be determined from the accelerometer data; and one of: in response to determining the monitoring device is attached to the patient, displaying health status information for the patient generated from the accelerometer data and the determined respiration rate data; or in response to determining the monitoring device is not attached to the patient, displaying an indication that the monitoring device is not attached to the patient.

In another aspect, a health status monitoring method includes: using a wireless monitoring device attached to a patient, measuring patient data; and using at least one electronic processor and as a function of time: analyzing the patient data to determine health status information for the patient including at least respiration rate data for the patient and storing the determined health status information for the patient; determining the monitoring device is no longer attached to the patient based at least on a determination that respiration rate data cannot be determined from the patient data; and in response to determining the monitoring device is no longer attached to the patient, storing an indication that the monitoring device is not attached to the patient.

One advantage resides in providing a reliable remote monitoring system for monitoring a patient.

Another advantage resides in reducing an amount of false alarms to treat a patient when the patient does not actually require treatment.

Another advantage resides in obtaining reliable vital signs of a patient from a wearable monitoring device.

Another advantage resides in accurately determining whether a patient is wearing a monitoring device.

Another advantage resides in dismissal of notifications and alarms received from sensors which are not being worn by a patient.

Another advantage resides in active tracing of patients not reliably wearing a sensor.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically shows a patient monitoring system in accordance with the present disclosure.
Fig. 2 shows an example flow chart of operations suitably performed by the system of Fig. 1.
Fig. 3 shows an example of a process performed by the system of Fig. 1.
Fig. 4 shows an example of an indication output by the system of Fig. 1.

### DETAILED DESCRIPTION

As used herein, the singular form of "a", "an", and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

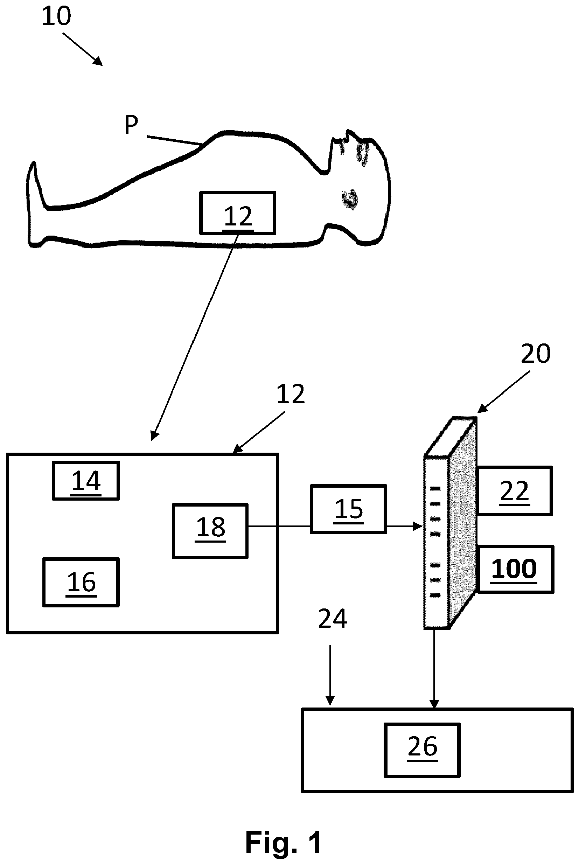

With reference to Fig. 1, a system **10** for monitoring an associated patient **P** is shown. As used herein, the term "patient" (and variants thereof) refers to, and includes, an outpatient, a discharged patient, a patient undergoing screening using the monitoring device as part of an annual medical checkup, or other person whose health condition is to be monitored. As shown in Fig. 1, the system **10** includes a monitoring device **12** that is wearable by, or otherwise attached to, the patient **P.** The monitoring device **12** can include any suitable monitoring device, such as a Healthdot^{®} device (available from Koninklijke Philips N.V., Eindhoven, the Netherlands), or a medical wearable device, a torso-worn vital signs health patch, a wrist-worn watches, a chest strap, a smart garment, medical ear buds/over the ear, a forehead or nose sensor, a smart ring, or so forth.

The monitoring device **12** can include a sensor **14** (such as an accelerometer **14** configured to measure patient data or, more specifically, accelerometer data **15,** or any other suitable data such as ECG data, EMG data, PPG data, and so forth), an on-board electronic processor **16,** and a wireless transmitter or transceiver **18** (referred to hereinafter as a transceiver **18**). In some embodiments, the electronic processor **16** is configured to collect the accelerometer data **15** from the accelerometer **14,** and the transceiver **18** is configured to transfer the accelerometer data **15** to a clinical health information system **20** operatively connected with the monitoring device via the transceiver **18.** As shown in Fig. 1, the clinical health information system **20** comprises a server computer with at least one electronic processor **22;** although the clinical health information system **20** can comprise any suitable computer device (e.g., a workstation computer, or a cellphone or smart tablet running an application ("app")). The clinical health information system **20** can be configured to perform a health status monitoring method **100** of monitoring the patient **P** by analyzing the accelerometer data **15.** In other embodiments, the on-board electronic processor **16** of the monitoring device **12** can perform the health status monitoring method **100.** In another embodiment, the clinical health information system **20** and the on-board electronic processor **16** can cooperatively perform the health status monitoring method **100.**

A consideration in design of the monitoring device **12** is battery life. For example, the Healthdot^{®} device is designed to be attached adhesively to skin of the subject in the region of the lower left rib, and to be worn continuously for up to about two weeks, remaining attached as the subject engages in normal activities such as showering. To maximize battery life, the Healthdot^{®} device employs a single sensor, namely an accelerometer, and the on-board electronic processor **16** derives a plurality of variables from the accelerometer data, namely activity, posture, heart rate (HR), and respiratory rate (RR). The Healthdot^{®} device extracts these variables from the accelerometer data using the on-board processor **16,** which facilitates integrating the Healthdot^{®} device with a commercial clinical health information system **20.** In other embodiments, it is contemplated to transmit the accelerometer data **15** as raw accelerometer data that is then processed by the clinical health information system **20** to derive the activity, HR, RR, and posture. Minimizing manufacturing cost is also a consideration. For example, the Philips Healthdot^{®} is a single-use device which is disposed of after use. As it contains only electronic devices and contacts only the exterior skin of the patient, the Healthdot^{®} device advantageously is not considered medical waste and can be disposed of as ordinary waste. In some examples, an adhesive of the Healthdot^{®} device can be disposed so that the sensor portion of the Healthdot^{®} device can be reused.

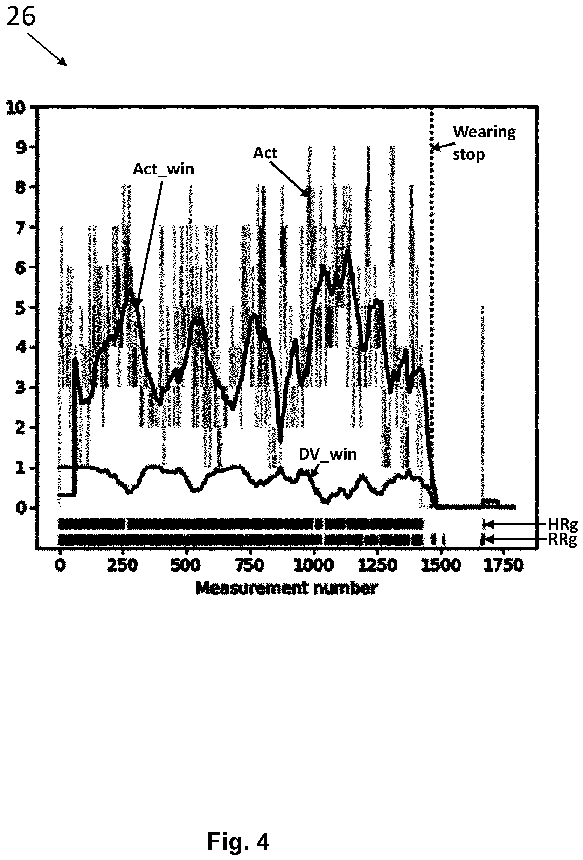

Fig. 4 shows an example of the indication **26** that the monitoring device **12** is not attached to the patient **P.** As shown in Fig. 4, a dashed line indicates when the monitoring device **12** is not being worn anymore. The importance of this feature is revealed by phantom values which appear in the accelerometer data **15,** which are caused by non-relevant accelerometric activity, such as data generated by an active monitoring device **12** which is not being worn anymore but instead is transported while disposed in the garbage bin. Without the wearing stop detection, the measurements after the dotted line may still result in an alarm and hence potentially costly actions from healthcare providers. With the system **10,** alarms based on these phantom values are prevented.

In Fig. 4, the bottom bar graphs labeled HRg and RRg indicate when valid HR and RR data, respectively, are collected. The "Act" data are the activity data. The DV win and ACT_win data are described below. Fig. 4 also indicates a "Wearing stop" line indicating when the monitoring device **12** stops being worn. However, it will be noted that occasional HR and RR measurements continue to be acquired after the "Wearing stop" point in time. These values could be the result of movement of the removed monitoring device **12.** As a nonlimiting illustrative example, if the activity monitor **12** is disposed of in a trashcan, then subsequent movement of the trashcan, for example as it is taken away and emptied, can result in occasional accelerometer data falsely indicative of a HR and/or RR measurement after the device **12** is no longer being worn.

The health status monitoring method **100** can be performed as a function of time, using data such as that plotted in Fig. 4, to accurately ascertain whether the monitoring device **12** is still being worn. Notably, embodiments of the method **100** can accurately determine when the monitoring device **12** stops being worn even in the presence of occasional accelerometer data falsely indicative of a HR and/or RR measurement after the device **12** is no longer being worn. In some embodiments disclosed herein, such determination is based at least in part on analysis of the extracted RR data, as it is recognized herein that false RR data are more likely to be generated than false extracted activity and/or HR data. For example, a trash can being carried by a person who is walking at a regular pace may produce movements at a rate of around 0.5-2 Hz (i.e., 30-120 strides per minute). This can be mistaken during the analysis of the extracted RR data for breathing, based on similarities in potential signal artifacts and overlapping frequency range. By contrast, while the heart rate frequency also has an overlapping frequency range, many HR analysis processes would filter out these artifacts based on shape dissimilarity. Hence, false RR measurements are likely to occur and generate alarms. In some embodiments, a combination of extracted HR and extracted RR are analyzed, and in some other embodiments extracted HR, extracted RR, and extracted activity data are analyzed, to further improve accuracy of the determination of whether the monitoring device **12** is still being worn.

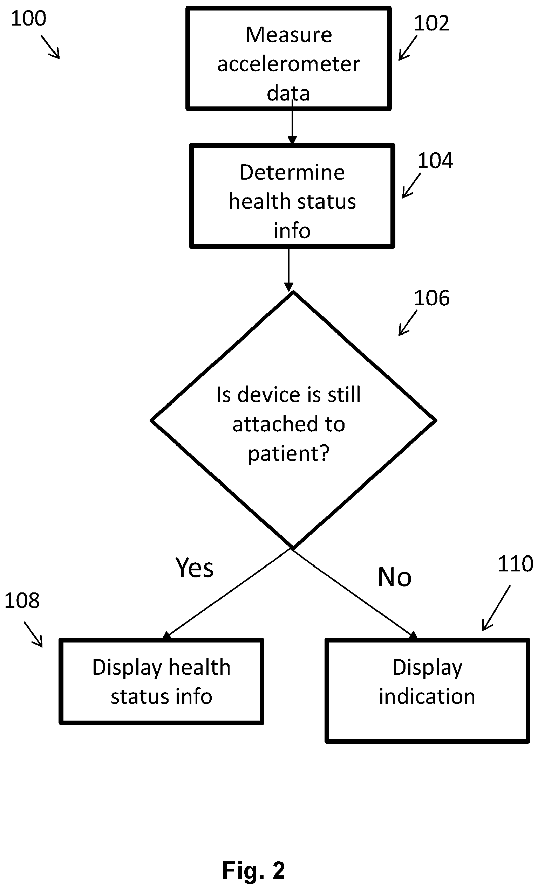

With reference now to Fig. 2, an illustrative embodiment of the health status monitoring method **100** is shown by way of a flowchart. At an operation **102,** the monitoring device **12** is attached to the patient **P,** and the accelerometer data **15** is measured by the accelerometer **14.**

At an operation **104,** the clinical health information system **20** and/or the on-board electronic processor **16** analyze the accelerometer data **15** to determine health status information for the patient. In some examples, the health status information can comprise respiration rate data for the patient **P,** or can comprise an indication that respiration rate data cannot be determined from the accelerometer data **15.** The determined health status information for the patient **P** can be stored at the clinical health information system **20.**

At an operation **106,** the clinical health information system **20** determines whether or not the monitoring device **12** is attached to the patient **P** based on, for example, at least a determination that respiration rate data cannot be determined from the accelerometer data **15.** To do so, in some embodiments, a running average of respiration rate values over a predefined sliding time window can be determined from the accelerometer data **15.** Each respiration rate value is indicative of respiration rate of the patient **P,** or a predetermined value that is not indicative of respiration rate of the patient **P.** The predefined sliding time window can include, for example, a range of 50 values and 60 values. The determination that respiration rate data cannot be determined from the accelerometer data **15** can also be based on a frequency of the predetermined values in the sliding time window of respiration rate values exceeding a predetermined threshold.

In some embodiments, in addition to respiration rate data, the analyzing operation **104** can determine heart rate data (or blood pressure) for the patient **P,** or an indication that heart rate data cannot be determined, from the accelerometer data **15.** The determining operation **106** can then include determining whether or not the monitoring device **12** is attached to the patient **P** is based on whether the analyzing operation determines that heart rate data cannot be determined from the accelerometer data **15.** To do so, in some embodiments, a running average of heart rate over the predefined sliding time window can be determined from the accelerometer data **15.** Each heart rate value is indicative of heart rate of the patient **P,** or a predetermined value that is not indicative of heart rate of the patient **P.** The determination that heart rate data cannot be determined from the accelerometer data **15** can also be based on a frequency of the predetermined values in the sliding time window of heart rate values exceeding a predetermined threshold.

In some embodiments, in addition to respiration rate data and/or heart rate data, the analyzing operation **104** can determine activity data (i.e., movement, posture, position, and so forth) for the patient **P,** or an indication that activity data cannot be determined, from the accelerometer data **15.** The determining operation **106** can then include determining whether or not the monitoring device **12** is attached to the patient **P** is based on whether the analyzing operation determines that activity data cannot be determined from the accelerometer data **15.** To do so, in some embodiments, a running average of activity values over the predefined sliding time window can be determined from the accelerometer data **15.** Each activity value is indicative of activity of the patient **P,** or a predetermined value that is not indicative of activity of the patient **P.** The determination that activity data cannot be determined from the accelerometer data **15** can also be based on a frequency of the predetermined values in the sliding time window of activity values exceeding a predetermined threshold.

Moreover, in addition to the accelerometer **14,** the monitoring device **12** can optionally include dedicated respiration sensors, and/or heart rate sensors to directly determine the respiration rate data and/or heart rate data, respectively (rather than deriving these vital signs from accelerometer data).

At an operation **108,** in response to the operation **106** determining that the monitoring device **12** is attached to the patient, health status information for the patient **P** generated from the accelerometer data **15** and the determined respiration rate data (and/or the determined heart rate data and/or the determined activity data) can be displayed on a display device **24** (e.g., a display of a workstation computer, a cellphone, a smart tablet, and so forth). Additionally or alternatively, in the operation **108** the determined respiration rate data (and/or the determined heart rate data and/or the determined activity data) can be stored in a patient electronic health or medical record (EHR or EMR) at the clinical health information system **20** for later retrieval or analysis by a clinician. Additionally or alternatively, in the operation **108** the determined respiration rate data (and/or the determined heart rate data and/or the determined activity data) can be analyzed to determine whether the patient is deteriorating, and if so the clinical health information system **20** outputs a suitable alarm to indicate the deterioration. For example, a nurses' station or other patient monitoring station can include a grid of status indicators for a set of monitored patients, and the alarm can be implemented as a flashing warning or other distinctive feature in the status indicator for the patient detected to be undergoing health deterioration. In some embodiments, in addition to respiration rate data, heart rate data, activity data, and posture data, other patient signals (e.g., blood pressure, SpO₂, temperature, etc.) can be extracted from the accelerometer data **15** and analyzed to determine the health status information for the patient **P.**

At an operation **110,** on the other hand, in response to the operation **106** determining that the monitoring device **12** is not attached to the patient **P,** an indication **26** at the monitoring device **12** is not attached to the patient **P** is displayed on the display device **24.** The indication **26** that the monitoring device **12** is not attached to the patient **P** can be stored at the clinical health information system **20.** Additionally or alternatively, in the operation **108** the record of determined respiration rate data (and/or heart rate data and/or the activity data) stored in the EHR or EMR at the clinical health information system **20** can record a suitable indicator that the monitoring device **12** is no longer being worn. For example, the indicator can include a flashing icon, an audible alarm, a visual message, and so forth. In this way, when a clinician later retrieves the data he or she is not misled by any occasional false HR and/or RR and/or activity data incorrectly measured after removal of the device **12.** Similarly, an analysis to determine whether the patient is deteriorating is not performed if the operation **106** determines that the monitoring device **12** is not attached to the patient **P.** (However, optionally the operation **110** may update the patient's status indicator in a nurses' station or other patient monitoring station to indicate that the monitoring device **12** is no longer being worn, so that a clinician can engage in appropriate follow-up such as contacting the patient to inquire as to where the monitoring device **12** is located). In another example, the indication **26** can be used for patient compliance during clinical trials, medication valuations, and so forth.

In some embodiments, the clinical health information system **20** and/or the on-board electronic processor **16** can control operation of the accelerometer **14.** For example, when the respiration rate and/or heart rate and/or activity values underrun the predetermined threshold, the accelerometer **14** can be deactivated, and thus prevented from acquiring additional accelerometer data **15** and, for example, saving battery life. When the respiration rate and/or heart rate and/or activity values exceed the predetermined threshold, the accelerometer **14** can be controlled to acquire additional accelerometer data **15.**

In some examples, the monitoring device **12** is used to collect patient data to derive an accurate and reliable indication about the patient deterioration. The monitoring device **12** can be used for other purposes such as activity monitoring, stamina evaluation, functional capacity evaluation, and so forth. The monitoring device **12** is equipped with the accelerometer **14,** with which body movements and physiological modalities are measured. Next to a characterization of patient deterioration or stability, this information can be used to detect whether or not the patient **P** is wearing the monitoring device **12.** The number of steps per day or an aggregate indicator of body movement and posture in relation to physiological modalities like heart rate and respiration rate are examples of the wearability indicators obtained from the monitoring device **12.** In addition, quality indicators on the measured vital signs can also give information about the monitoring device **12** being worn or not, as the underlying raw signals may be quite different if the monitoring device **12** is worn or not.

The clinical health information system **20** is configured to process wearability data and advise clinical practitioners on the appropriateness of responding and acting to any alarm or notification coming from the monitoring device **12.** The clinical health information system **20** evaluates wearability and consequently notifies the hospital caregiver when a patient **P** is active but seems to be not wearing the monitoring device **12** anymore. The hospital caregiver can subsequently remove the patient **P** from their active surveillance database or contact the patient **P** if this is an unexpected removal of the monitoring device **12.**

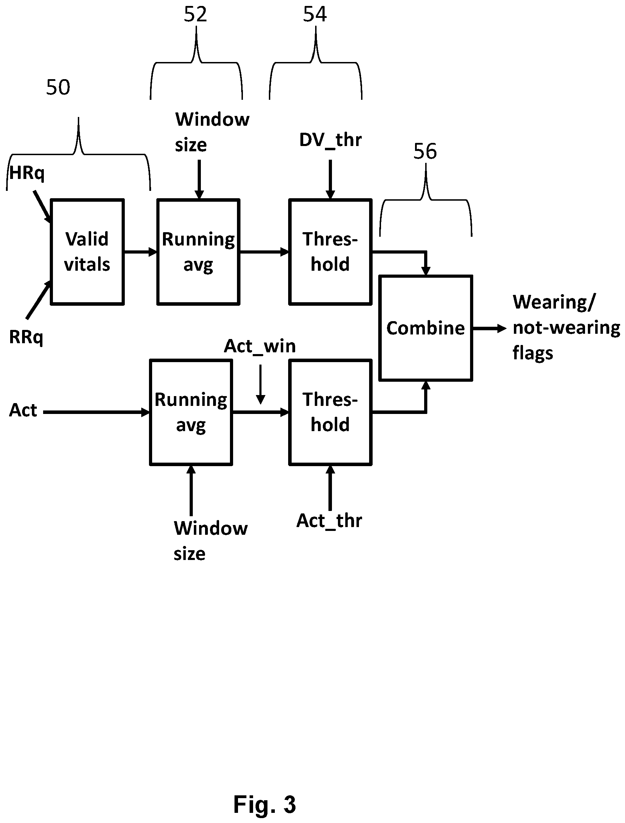

Fig. 3 shows a schematic representation of an example of the determination operation **106.** The accelerometer **14** is configured to obtain information about the quality of the heart rate (HRq) and respiration rate (RRq) data, and activity (Act) in the accelerometer data **15.** The accelerometer data **15** can arrive at an interval of, for example, about 5 minutes.

At an operation **50,** a determination is made, for each time point in the accelerometer data **15,** whether HRq and RRq are both valid. HRq and RRq are binary valued, indicating whether HR and RR measurements are valid or not. This is indicated by a value DV = 1 if so, or DV = 0 if either or both vitals are of too low quality. Act is represented as a discrete value on a scale from 0 (no activity) to 10 (high activity).

At an operation **52,** a running average is taken over a predefined sliding window, of both the combined validity DV and the activity level Act, which can yield running averages DV_win and Act_win, respectively. The window size can, for example, be equal to 50 (or 60); 12 per hour, in some nonlimiting illustrative examples. This is minimally equal to 5 hours, in some nonlimiting illustrative embodiments. The window size is changeable, and a balance needs to be struck between being strict with all detection being true detections (high specificity) or being less strict with increased chance of false detection (high sensitivity).

At an operation **54,** thresholds of DV_thr = 0.3 and Act_thr = 1 are applied, and a determination is made where both DV_win < DV thr and Act_win < Act_thr. Here, the first 49 values of DV_win and Act_win can be discarded, as they are not based on a full window of, for example, 50 measurements yet (a run-in effect). Optionally, the first time points can be discarded until threshold DV thr or threshold DV Act is exceeded (or both), to ensure that we are passed a run-in period. The thresholds can be set differently for other sensors, pending the use case and sensor accuracy/sensitivity.

At an operation **56,** at the time points where DV_win < DV thr and Act_win < Act thr, the determination is made as to whether the monitoring device **12** is not worn. The determination is made to conclude that the monitoring device **12** is not worn from the very first time point that this happens, as it typically cannot be re-applied.

In some embodiments, quality indicators of the vital signs may be determined, and this may be combined with the actual measurements to determine the moments when they are valid. Furthermore, the determination of whether the vital signs are valid may include the activity information, as well as other information such as posture info coming from the monitoring device **12.**

In some embodiments, the wearing stop time point can be refined. For instance, it may be chosen to go back in time from the detected wearing stop time point (i.e., the dotted line in Fig. 4) to the last time point with valid values for HR and RR, and label that as the wearing stop. Optionally, the period between these time points may be labelled as 'uncertain.'

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A system **(10)** comprising:
a monitoring device **(12)** including an accelerometer **(14),** an on-board electronic processor **(16),** and a wireless transmitter or transceiver **(18),** the monitoring device configured to be attached to a patient and the accelerometer configured to measure accelerometer data **(15);** and
a clinical information system **(20)** including at least one electronic processor **(22),** the clinical information system operatively connected with the monitoring device via the wireless transmitter or transceiver of the monitoring device;
wherein the on-board electronic processor of the monitoring device and the clinical information system cooperatively perform a health status monitoring method **(100)** including:
analyzing the accelerometer data to determine respiration rate data for the patient or an indication that respiration rate data cannot be determined from the accelerometer data;
determining whether the monitoring device is attached to the patient based at least on the whether the analyzing determines that respiration rate data cannot be determined from the accelerometer data; and
one of:
in response to determining the monitoring device is attached to the patient, displaying health status information for the patient generated from the accelerometer data and the determined respiration rate data; or
in response to determining the monitoring device is not attached to the patient, displaying an indication **(26)** that the monitoring device is not attached to the patient.

2. The system **(10)** of claim 1, wherein:
the analyzing the accelerometer data **(15)** further determines heart rate data for the patient or an indication that heart rate data cannot be determined from the accelerometer data; and
the determining of whether the monitoring device **(12)** is attached to the patient is further based on whether the analyzing determines that heart rate data cannot be determined from the accelerometer data.

3. The system **(10)** of either one of claims 1 and 2, wherein:
the analyzing the accelerometer data **(15)** further determines activity data for the patient or an indication that activity data cannot be determined from the accelerometer data; and
the determining of whether the monitoring device **(12)** is attached to the patient is further based on whether the analyzing determines that activity data cannot be determined from the accelerometer data.

4. The system **(10)** of claim 1, wherein the analyzing of the accelerometer data **(15)** to determine respiration rate data for the patient or an indication that respiration rate data cannot be determined from the accelerometer data includes:
determining a running average of respiration rate values over a predefined sliding time window from the accelerometer data, each respiration rate value being indicative of respiration rate of the patient or a predetermined value that is not indicative of respiration rate of the patient; and
determining that respiration rate data cannot be determined from the accelerometer data based on a frequency of the predetermined values in the sliding time window of respiration rate values exceeding a threshold.

5. The system **(10)** of claim 4, wherein:
the analyzing further includes determining heart rate data for the patient or an indication that heart rate data cannot be determined from the accelerometer data **(15)** by operations including:
determining a running average of heart rate values over a predefined sliding time window from the accelerometer data, each heart rate value being indicative of heart rate of the patient or a predetermined heart rate value that is not indicative of heart rate of the patient; and
determining that heart rate data cannot be determined from the accelerometer data if a frequency of the predetermined heart rate values in the predefined sliding time window of heart rate values exceeding a second threshold; and
the determining of whether the monitoring device **(12)** is attached to the patient is further based on whether the analyzing determines that heart rate data cannot be determined from the accelerometer data.

6. The system **(10)** of either one of claims 4 and 5, wherein:
the analyzing further includes determining activity data for the patient or an indication that activity data cannot be determined from the accelerometer data **(15)** by operations including:
determining a running average of activity values over a predefined sliding time window from the accelerometer data, each activity value being indicative of activity of the patient or a predetermined activity value that is not indicative of activity of the patient; and
determining that activity data cannot be determined from the accelerometer data if a frequency of the predetermined activity values in the predefined sliding time window of activity values exceeding a second threshold; and
the determining of whether the monitoring device is attached to the patient is further based on whether the analyzing determines that activity data cannot be determined from the accelerometer data.

7. The system **(10)** of any one of claims 4-6, wherein the predefined sliding time window comprises a range of 50-60 values.

8. A health status monitoring method **(100)** comprising:
using a wireless monitoring device **(12)** attached to a patient, measuring patient data **(15);** and
using at least one electronic processor **(16, 22)** and as a function of time:
analyzing the patient data to determine health status information for the patient including at least respiration rate data for the patient and storing the determined health status information for the patient;
determining the monitoring device is no longer attached to the patient based at least on a determination that respiration rate data cannot be determined from the patient data; and
in response to determining the monitoring device is no longer attached to the patient, storing an indication that the monitoring device is not attached to the patient.

9. The health status monitoring method **(100)** of claim 8, further comprising, as a function of time:
displaying the determined health status information for the patient on a display **(24);** and
in response to the determination that the monitoring device **(12)** is no longer attached to the patient, displaying an indication that the monitoring device is no longer attached to the patient.

10. The health status monitoring method **(100)** of either one of claims 8 and 9, further comprising, using at least one electronic processor **(16, 22)** and as a function of time:
determining heart rate data for the patient from the patient data **(15)** wherein the health status information for the patient further includes the heart rate data;
wherein the determining that the monitoring device **(12)** is no longer attached to the patient is further based on a determination that heart rate data cannot be determined from the patient data.

11. The health status monitoring method **(100)** of any one of claims 8-10, further comprising, using at least one electronic processor **(16, 22)** and as a function of time:
determining activity data for the patient from the patient data **(15)** wherein the health status information for the patient further includes the activity data;
wherein the determining that the monitoring device **(12)** is no longer attached to the patient is further based on a determination that activity data cannot be determined from the patient data.

12. The health status monitoring method **(100)** of claim 8, wherein:
the analyzing of the patient data **(15)** to determine the respiration rate data for the patient includes determining a running average of respiration rate values over a predefined sliding time window from the patient data, each respiration rate value being indicative of respiration rate of the patient or a predetermined value that is not indicative of respiration rate of the patient; and
the determination that respiration rate data cannot be determined from the patient data is based on a frequency of the predetermined values in the predefined sliding time window of respiration rate values exceeding a threshold.

13. The health status monitoring method **(100)** of claim 12, further comprising, using at least one electronic processor **(16, 22)** and as a function of time:
analyzing the patient data **(15)** to determine heart rate data for the patient, the health status information further including the heart rate data, the determining of the heart rate data including determining a running average of heart rate values over a predefined sliding time window from the patient data, each heart rate value being indicative of heart rate of the patient or a predetermined heart rate value that is not indicative of heart rate of the patient;
determining that heart rate data cannot be determined from the patient data based on a frequency of the predetermined heart rate values in the predefined sliding time window of heart rate values exceeding a second threshold;
wherein the determining that the monitoring device **(12)** is no longer attached to the patient is further based on the determination that heart rate data cannot be determined from the patient data.

14. The health status monitoring method **(100)** of either one of claims 12 and 13, further comprising, using at least one electronic processor **(16, 22)** and as a function of time:
analyzing the patient data **(15)** to determine activity data for the patient, the health status information further including the activity data, the determining of the activity data including determining a running average of activity values over a predefined sliding time window from the patient data, each activity value being indicative of activity of the patient or a predetermined activity value that is not indicative of activity of the patient;
determining that activity data cannot be determined from the patient data based on a frequency of the predetermined activity values in the predefined sliding time window of activity values exceeding a second threshold;
wherein the determining that the monitoring device **(12)** is no longer attached to the patient is further based on the determination that activity data cannot be determined from the patient data.

15. The health status monitoring method **(100)** of any one of claims 8-14, wherein the patient data **(15)** comprises accelerometer data acquired by an accelerometer **(14)** of the wireless monitoring device **(12).**
